# EUROPEAN PATENT SPECIFICATION

(11) **EP 4 470 468 B1**
(45) Date of publication and mention of the grant of the patent: **19.08.2026**
(21) Application number: 23176931.6
(22) Date of filing: 02.06.2023
(51) Int. Cl.: A61B 5/349

(54) **SYSTEM FOR IDENTIFYING A CARDIAC ARRHYTHMIA EVENT FOLLOWING A MYOCARDIAL INFARCTION**
SYSTEM ZUR IDENTIFIZIERUNG EINES HERZARRHYTHMIEEREIGNISSES NACH EINEM MYOKARDINFARKT
SYSTÈME D'IDENTIFICATION D'UN ÉVÉNEMENT D'ARYTHMIE CARDIAQUE APRÈS UN INFARCTUS DU MYOCARDE

(43) Date of publication of application: 04.12.2024
(73) Proprietor: BIOTRONIK SE & Co. KG, 12359 Berlin (DE)
(72) Inventor: PARISH, Patrick L., Portland, 97219 (US); MUESSIG, Dirk, West Linn, 97068 (US); WHITTINGTON, R. Hollis, Portland, 97202 (US); TAFF, Brian M., Portland, 97217 (US)
(74) Representative: Biotronik Corporate Services SE

(56) References cited:
- US-A- 6 047 206
- US-A1- 2010 256 701
- US-A1- 2011 295 333
- US-A1- 2012 179 055

## Description

The present invention generally relates to a system for identifying a cardiac arrhythmia event in a patient and to a method for operating such a system.

A system of the type concerned herein comprises an implantable medical device for sensing electrocardiogram signals, the implantable medical device comprising an arrangement of electrode poles configured to sense electrocardiogram signals. A processing arrangement is configured for processing electrocardiogram signals obtained by the implantable medical device.

An implantable medical device of this kind may for example be a pacemaker, an implantable cardioverter defibrillator, a sensor device such as a bio-sensor, or a monitoring device. The implantable medical device herein is configured to sense electrocardiogram signals.

For example, the implantable medical device may be a monitoring device which is configured to record electrocardiogram signals and to communicate recorded electrocardiogram signals or information derived from recorded electrocardiogram signals to an external device in the context of a home monitoring system.

US 6,047,206 A describes techniques for generating localized cardiac measures for use in detecting a myocardial infarction (i.e., a heart attack). The techniques of the also may be used to identify the blocked artery or arteries, and to track the progression of an evolving myocardial infarction, with respect to factors such as the transition from ischemia to scarring, the degree of scarring, and the efficacy of thrombolysis or other treatments.

An implantable medical device as for example described in EP 3 278 836 B1 may for example comprise a housing and an arrangement of electrode poles arranged on the housing. The electrode poles herein are arranged on the housing of the implantable medical device such that the electrode poles are aligned along a longitudinal axis along which the implantable medical device extends. The electrode poles may for example be formed by housing segments which are made from an electrically conductive material such as a metal material and are exposed to the outside such that they may be brought into electrical contact with surrounding tissue in order to establish an electrical coupling to the tissue in an implanted state of the implantable medical device.

An implantable medical device as e.g. used in a home monitoring system shall allow for a reliable monitoring of a physiological state of a patient. In particular, using the implantable medical device it shall be possible to reliably detect an abnormal cardiac state based on recorded electrocardiogram signals. If an abnormality is detected in electrocardiogram signals, the implantable medical device shall be enabled to communicate with for example an external device of a home monitoring system, e.g. in order to trigger a message to a service center to alert medical personnel of a potential need for attention.

A system and method of the type concerned herein shall in particular be configured for identifying cardiac arrhythmia events in a patient. Generally, within a system, cardiac arrhythmia events such as ventricular tachycardia events, ventricular fibrillation events or premature ventricular contraction events are identified by processing electrocardiogram signals according to an amplitude and frequency content of the processed signals. Within detection schemes for identifying cardiac arrhythmia events, which are posed with the inherent problem to distinguish relevant signal portions from noise contributions, detection settings generally are prone to a conflict between on the one hand allowing for an identification of cardiac arrhythmia events with a high sensitivity and on the other hand avoiding a large number of false positive classifications, which may increase the review burden and alarm fatigue for clinical professionals.

In particular, if cardiac arrhythmia events shall be detected with high sensitivity based on electrocardiogram signals, this typically comes at the expense of an increased number of false positive classifications. If however the sensitivity is reduced in order to reduce the number of false positive classifications, this comes at the expense of a potentially less reliable detection of cardiac arrhythmia events.

The risk of cardiac arrhythmia events for a patient may depend on the physical state, in particular the cardiac state, of the patient. In particular, during the acute phase following a myocardial infarction event, a patient may have an increased risk of ventricular tachycardia and premature ventricular contractions. If the patient is in a state of myocardial infarction, cardiac arrhythmia events may more likely occur and may have a more severe impact on the patient as compared to a healthy cardiac state of the patient.

The invention is defined in the appended claims. Aspects, embodiments and examples disclosed herein which do not fall within the scope of the appended claims do not form part of the invention, and are merely provided for illustrative purposes.

It is an objective to provide a system for identifying cardiac arrhythmia events in a patient which in a reliable manner allow for the identification of cardiac arrhythmia events based on electrocardiogram signals by taking a physical state of the patient into account.

In one aspect, a system for identifying a cardiac arrhythmia event in a patient comprises an implantable medical device for sensing electrocardiogram signals, the implantable medical device comprising an arrangement of (preferably two) electrode poles configured to sense electrocardiogram signals. The system further comprises a processing arrangement for processing electrocardiogram signals obtained by the implantable medical device. The processing arrangement is configured to use a first setting of at least one detection parameter for detecting a cardiac arrhythmia event based on said electrocardiogram signals, obtain information indicative of a myocardial infarction event, and in response to obtaining said information, adapt said at least one detection parameter to a second setting different than the first setting for detecting a cardiac arrhythmia event in a time period subsequent to obtaining said information indicative of a myocardial infarction event.

In general, the system is configured to identify a cardiac arrhythmia event based on electrocardiogram signals as sensed by means of the implantable medical device. Electrocardiogram signals herein are processed, and based on the processing a cardiac arrhythmia event, such as a ventricular tachycardia (VT) event, a ventricular fibrillation (VF) event or a premature ventricular contraction (PVC) event, is identified. Other arrhythmias are also relevant and include supraventricular tachycardia (SVT) events, bradycardia, asystole, sudden rate changes, ectopic events, and other morphological changes to the ECG such as ST segment elevation or depression, changes in timing between components of the ECG, and other diagnostic characteristics. These arrhythmias may be also considered as a cardiac arrhythmia event (in all described embodiments).

The identification herein takes place by assessing the electrocardiogram signals according to one or multiple detection parameters, for example by employing certain filter settings, settings for autosensing parameters, timer settings or settings for refractory periods, each of these parameters representing a detection parameter used for identifying cardiac arrhythmia events.

For example, a cardiac arrhythmia event such as a premature ventricular contraction event may be detected based on a timing by assessing a timing distance of at least one ventricular contraction event with respect to a prior ventricular contraction event and a subsequent ventricular contraction event. The setting of a timing threshold for a timing distance with respect to the prior ventricular contraction event and/or with respect to a subsequent ventricular contraction event determines the sensitivity by which a premature ventricular contraction event is identified based on a processing of electrocardiogram signals.

**In** another example, a ventricular tachycardia event may be identified based on a timing of a sequence of heartbeats. Herein, a ventricular tachycardia event may for example be identified if for a predefined number of heartbeats the heart rate is above a predefined heart rate threshold. The predefined number of heartbeats and the predefined heart rate threshold herein represent detection parameters.

In a default state of a patient, that is a state in which no actual or probable myocardial infarction event has been identified in a patient, a setting for one or multiple detection parameters may be employed which allows for a detection of a cardiac arrhythmia event with a suitable sensitivity at a low risk of false positive classifications. Accordingly, in a default state of a patient the processing arrangement of the system is configured to use a first setting of at least one detection parameter for detecting a cardiac arrhythmia event based on sensed electrocardiogram signals.

However, in case information about a myocardial infarction event is obtained - that is information indicating that a myocardial infarction event has already occurred or very likely will in the very near future occur - the processing arrangement is configured to adapt the at least one detection parameter to a second setting different than the first setting for detecting a cardiac arrhythmia event in a time period subsequent to the obtaining of the information about the myocardial infraction event. In case a myocardial infarction event is identified (either automatically by the system or by user input), hence, a setting for detection of a cardiac arrhythmia event is adapted, such that within a time period following the detection of a myocardial infarction event cardiac arrhythmia events may be detected e.g. with increased sensitivity, at the expense of a potentially increased number of false positive classifications within the time period.

As in an acute phase following a myocardial infraction event a patient may exhibit an increased risk of cardiac arrhythmias, in particular ventricular tachycardias, ventricular fibrillations or premature ventricular contractions, within the time period following the identification of a myocardial infarction event it may be desirable to detect cardiac arrhythmias with an increased sensitivity, an increased number of false positive classifications potentially being acceptable within that time period of increased alert subsequent to a myocardial infarction event.

For example, for detecting a premature ventricular contraction event in the time period following the identification of a myocardial infarction event a setting for one or multiple timing thresholds for assessing a timing distance of at least one ventricular contraction event with respect to a prior ventricular contraction event and with respect to a subsequent ventricular contraction event may be adapted, such that at least one ventricular contraction event in question is classified with an increased sensitivity as a premature ventricular contraction event.

In another example, for detecting a ventricular tachycardia a setting for the number of heartbeats or a setting for a heart rate threshold may be adapted. For example, in a default state for example a sequence of heartbeats may be classified as a ventricular tachycardia if e.g. for a predefined number of heartbeats (e.g. a number between 6 to 20 heartbeats, e.g. 8 heartbeats) a predefined heart rate threshold is exceeded (e.g. in a range between 150 bpm and 220 bpm, e.g. 200 bpm). Following the identification of a myocardial infarction event, the settings may be adapted, for example by reducing the predefined number of heartbeats (e.g. to a number between 2 and 10 heartbeats, e.g. 4 heartbeats) and/or the heart rate threshold (for example to a value between 80 bpm and 140 bpm). Whereas in a default state for example a sequence of heartbeats may be classified as a ventricular tachycardia if e.g. for 8 heartbeats a predefined heart rate threshold of 200 bpm is exceeded, the setting may be adapted following the identification of a myocardial infraction event such that a sequence of heartbeats is classified already as a ventricular tachycardia if e.g. for 4 heartbeats a predefined heart rate threshold of e.g. 120 bpm is exceeded. By reducing the value for the number of heart beats and/or the heart rate threshold, hence, the sensitivity for detecting a ventricular tachycardia is increased.

In one embodiment, the implantable medical device comprises a processing module forming at least part of the processing arrangement. In particular, in one embodiment the processing module may be configured to carry out steps of using a first setting of at least one detection parameter for detecting a cardiac arrhythmia event based on said electrocardiogram signals, of obtaining information indicative of a myocardial infarction event, and of adapting, in response to obtaining said information, said at least one detection parameter to a second setting different than the first setting for detecting a cardiac arrhythmia event in a time period subsequent to obtaining information about a myocardial infarction event. In another embodiment, the processing module may be configured to carry out only some, but not all of the processing. For example, the processing module of the implantable medical device may be configured to identify ventricular contraction events and to record a waveform relating to ventricular contraction events, wherein the actual processing to identify a cardiac arrhythmia event is carried out by another processing entity, for example of an external device which is in communication with the implantable medical device, for example within the context of a home monitoring system. In yet another embodiment, the implantable medical device may be used to only record electrocardiogram signals, wherein the entire processing for identifying cardiac arrhythmia events is carried out externally to the implantable medical device, for example by an external device within the context of a home monitoring system.

In one embodiment, the processing arrangement is configured, for obtaining the information indicative of a myocardial infarction event, to detect a myocardial infarction event based on sensed electrocardiogram signals. The detection of a myocardial infarction event hence is carried out by the processing arrangement, for example by a processing module of the implantable medical device. A myocardial infarction event hence is automatically detected by processing sensed electrocardiogram signals, for example by assessing a so-called ST segment elevation in QRS complexes of electrocardiogram signals.

In another embodiment, information relating to a myocardial infarction event may be input to the system, for example by a manual user input, such that information relating to the occurrence of a myocardial infarction event is input manually by a clinical user, for example into an external device which is in communication connection with the implantable medical device of the system.

In one embodiment, the processing arrangement is configured to adapt the at least one detection parameter in response to the myocardial infarction event to detect a cardiac arrhythmia event with an increased sensitivity in the time period subsequent to obtaining the information indicative of a myocardial infarction event. The settings of one or multiple detection parameters, for example relating to timing thresholds for detecting premature ventricular contraction events or filter settings for example for detecting ventricular fibrillation or ventricular tachycardia events, are adapted in response to obtaining information with respect to the occurrence of a myocardial infraction event such that a detection of a cardiac arrhythmia event takes place with an increased sensitivity.

In one embodiment, the processing arrangement is configured to revert the at least one detection parameter slowly back to the default state (before myocardial infarction event). In other words, slowly revert back the at least one detection parameter toward the default state after the end of the time period of increased sensitivity (as opposed to a sudden, rapid change). This includes three options: 1) automatically revert after a programmable time period, 2) revert upon reaching some condition of the signal, like ST segment elevation goes away, and 3) adaptively revert back to original/default state based on some criteria (for example, lack of subsequent increased arrhythmias).

In one embodiment, the processing arrangement is configured to detect, as a cardiac arrhythmia event, at least one of a ventricular tachycardia (VT) event, a ventricular fibrillation (VF) event, and asystole event, a bradycardia event and/or a premature ventricular contraction (PVC) event. For example, the at least one detection parameter includes at least one of a threshold for a heart rate value, a number of heartbeats for determining a heart rate value, a timing threshold indicative of a timing between successive heartbeats, or an amplitude threshold. In one embodiment, the at least one detection parameter may include at least one of a filter setting, a setting of autosensing parameters, timer settings, or settings for refractory periods.

In one embodiment, the processing arrangement is configured to adapt the at least one detection parameter back to the first setting for detecting a cardiac arrhythmia event upon lapse of the time period. Within the time period following a myocardial infarction event a second setting for the at least one detection parameter is used. Upon lapse of the time period, the system automatically reverts back to the default state such that upon lapse of the time period again the first setting for the at least one detection parameter is used for identifying a cardiac arrhythmia event. Upon lapse of the time period, hence, previous, default settings for one or multiple detection parameters are automatically restored, such that cardiac arrhythmia events are for example detected with a (slightly) reduced sensitivity at a reduced risk of false positive classifications.

The time period may for example be in a range between 10 minutes and 30 days, preferably between 1 hour and 30 days, particularly preferably between 1 hour and 24 hours. The time period may in particular span a time range covering an acute phase following a myocardial infarction event.

In one embodiment, the processing arrangement is configured to identify at least one ventricular contraction event (or a series of ventricular contraction events) based on electrocardiogram signals and to classify the ventricular contraction event (or at least a part of the series of ventricular contraction events) as a cardiac arrhythmia event (or a series containing at least one cardiac arrhythmia event) based on the at least one detection parameter. Based on sensed electrocardiogram signals hence ventricular contraction events, in particular represented by QRS wave forms in the electrocardiogram signals, are identified and are assessed in order to classify a particular ventricular contraction event or a sequence of ventricular contraction events as a cardiac arrhythmia event, such as a ventricular tachycardia, a ventricular fibrillation or a premature ventricular contraction event.

In one embodiment, the processing arrangement is configured to classify at least one ventricular contraction event as a premature ventricular contraction event based on a first timing distance between the ventricular contraction event and an immediately prior ventricular contraction event and/or a second timing distance between the ventricular contraction event and an immediately subsequent ventricular contraction event. Generally, it is characteristic for a premature ventricular contraction event that the contraction event comes at a relatively short timing distance after a prior ventricular contraction event and is followed by a lengthy pause before another, regular ventricular contraction event occurs. Hence, the timing of the currently assessed ventricular contraction event may be evaluated in order to define an additional, necessary condition which must be fulfilled in order to classify the currently assessed ventricular contraction event as a premature ventricular contraction event. If for example the first timing distance between the ventricular contraction event and the immediately prior ventricular contraction event is smaller than a first timing threshold and/or the second timing distance between the ventricular contraction event and the immediately subsequent ventricular contraction event is larger than a second timing threshold, and if in addition the assessment of at least one discrimination metric value computed based on the currently assessed ventricular contraction event yields an abnormality, the currently assessed ventricular contraction event may be classified as a premature ventricular contraction event.

In one embodiment, the first timing threshold represents a first detection parameter, and/or the second timing threshold represents a second detection parameter. For at least one of the detection parameters in a default state a first setting for the detection parameter is used, for example a first setting for the first timing threshold. Upon the occurrence of a myocardial infarction event the processing arrangement is configured to adapt a particular detection parameter such that in a time period following the myocardial infarction event a second setting for the particular detection parameter is used. Hence, settings for the first timing threshold and/or the second timing threshold may be adapted following the detection of a myocardial infarction event.

In one embodiment, in addition to classifying at least one ventricular contraction event as a premature ventricular contraction event based on timing distances, the processing arrangement may be configured to identify a premature ventricular contraction event based on a morphology of a wave form relating to a ventricular contraction event. In particular, in one embodiment, the processing arrangement may be configured to compute at least one discrimination metric value for a ventricular contraction event, to compare said at least one discrimination metric value to at least one of a first reference value computed based on a first number of prior ventricular contraction events and a second reference value computed based on a second number of subsequent ventricular contraction events, and to classify said ventricular contraction event as a premature ventricular contraction event based on said comparison.

For identifying a premature ventricular contraction event representing a potentially hazardous cardiac arrhythmia event, it in particular may be made use of the fact that for a premature ventricular contraction event it can be assumed that a waveform relating to the premature ventricular contraction event comprises an abnormal shape, in comparison to other, regular ventricular contraction events. Hence, within the system, a premature ventricular contraction event may be identified by assessing the morphology of a waveform relating to a particular ventricular contraction event. If it is found that abnormalities exist in a particular ventricular contraction event indicating a premature ventricular contraction event, the particular ventricular contraction event in question may be classified as a premature ventricular contraction event.

Within the system, one or multiple discrimination metric values may be computed. The discrimination metric values relate to the morphology of a waveform of the ventricular contraction event which is currently assessed. The one or the multiple discrimination metric values are compared to reference values, and based on the comparison it is identified whether the discrimination metric values indicate an abnormal waveform possibly indicative of a premature ventricular contraction event (or not).

Herein, the one or the multiple discrimination metric values are compared to one or multiple reference values relating to prior ventricular contraction events, which generally can be assumed to be regular ventricular contraction events of regular heartbeats, and/or to one or multiple reference values relating to subsequent ventricular contraction events, which also generally can be assumed to relate to regular heartbeats. The reference values hence are determined based on prior ventricular contraction events and/or subsequent ventricular contraction events.

Each reference value should indicate a value for a discrimination metric value in question which is indicative of a normal state and hence a normal ventricular contraction event of a regular heartbeat. If, by the comparison of the discrimination metric value computed for the instant ventricular contraction event, it is found that the discrimination metric value differs from the reference value e.g. by more than a certain margin, it is identified that an abnormal waveform exists exhibiting an abnormal morphology, such that the ventricular contraction event may be classified as a premature ventricular contraction event.

The first number of prior ventricular contraction events, based on which the first reference value is determined, may for example lie in a range between 2 to 50, for example 3 to 20, for example 6 prior ventricular contraction events. The prior ventricular contraction events may be successive ventricular contraction events immediately prior to the instantly assessed ventricular contraction event, or may be non-successive.

The second number of subsequent ventricular contraction events, based on which the second reference value is determined, may for example lie in a range between 2 to 50, for example 3 to 20, for example 6 subsequent ventricular contraction events. The subsequent ventricular contraction events may be successive ventricular contraction events immediately subsequent to the instantly assessed ventricular contraction event, or may be non-successive.

The first number may be equal to the second number or may differ from the second number.

The classification of a premature ventricular contraction event generally takes place, in particular if a second reference value relating to subsequent ventricular contraction events is taken into account, with a time delay to cover a time period within which the subsequent ventricular contraction events are recorded.

In one embodiment, the processing arrangement is configured to compute the at least one discrimination metric value, with relation to a signal portion relating to the ventricular contraction event, based on at least one of the following: a maximum positive amplitude, a maximum negative amplitude, a maximum rectified amplitude, a peak-to-peak amplitude, a maximum first derivative value, a maximum second derivative value, an area value under a curve of said signal portion until a first zero-crossing, an area value under a curve of said signal portion between a first zero-crossing and a second zero crossing, an area value under a curve of said signal portion between a second zero-crossing and a third zero crossing, a time duration value until a first zero-crossing, a time duration value between a first zero-crossing and a second zero crossing, a time duration value between a second zero-crossing and a third zero crossing and/or a time duration value between an upward crossing and a downward crossing of a ventricular detection threshold.

One or multiple quantities may be computed, accordingly, as one or multiple discrimination metric values. The discrimination metric value may for example be computed according to the maximum positive amplitude, the maximum negative amplitude, a maximum rectified amplitude, or a peak-to-peak amplitude of a waveform relating to a currently assessed ventricular contraction event. Alternatively or in addition, a discrimination metric value may be computed according to a maximum value of a first derivative or a second derivative of the waveform relating to the currently assessed ventricular contraction event. Alternatively or in addition, the discrimination metric value may be computed according to an area value indicative of an area under a curve relating to the currently assessed ventricular contraction event, wherein the area value may relate to the area until the first zero-crossing, to an area between the first zero-crossing and a second zero-crossing, or to an area between the second zero-crossing and a third zero-crossing. Alternatively or in addition, the discrimination metric value may be computed to be indicative of a time duration, for example between the initial detection of the ventricular contraction event and a first zero-crossing, between the first zero-crossing and a second zero-crossing, or between the second zero-crossing and a third zero-crossing, or between an upward crossing of a ventricular detection threshold, based on which the ventricular detection event is initially identified, and a downward crossing of the ventricular detection threshold.

Generally, one or multiple discrimination metric values may be computed, wherein any combination of discrimination metric values may be used to identify a premature ventricular contraction event. Each discrimination metric value herein is compared to at least one of an associated first reference value and an associated second reference value, the particular reference value being indicative of a normal value for the particular discrimination metric value in question.

Each reference value may be computed by applying a statistical analysis of prior ventricular contraction events and/or subsequent ventricular contraction events. In particular, the first reference value may be computed based on a first statistical measure relating to the first number of prior ventricular contraction events. In turn, the second reference value may be computed based on a second statistical measure relating to the second number of subsequent ventricular contraction events. The first reference value hence is determined by statistical analysis of prior ventricular contraction events. In contrast, the second reference value is determined by statistical analysis of subsequent ventricular contraction events.

The particular reference value in particular may be computed according to any standard statistical quantity obtained by statistical analysis. For example, the first reference value and/or the second reference value may be computed according to a mean value, a standard deviation value, a coefficient of variation, a Shannon entropy value, an exponential moving average value e.g. according to a function with varying beats/varying weights associated with the surrounding beats, a median value, a percentile value, e.g. a 5% to 95 % percentile value, a skew value, a kurtosis value, and/or a root mean square value e.g. of successive differences. If for example the discrimination metric value in question is computed according to the maximum amplitude of the waveform relating to the current ventricular contraction event, the reference value is determined by e.g. an averaging or by another statistical measure of the maximum amplitude values of prior ventricular contraction events and/or subsequent ventricular contraction events. If the discrimination metric value in question is an area value relating to the waveform of the current ventricular contraction event or a time duration value, the reference value is determined by a statistical measure relating to the associated area value or time duration value of prior ventricular contraction events and/or subsequent ventricular contraction events.

The particular reference value may be computed, in a default state, by using a first setting, for example according to a default statistical measure. In response to the occurrence of a myocardial infarction event, the particular reference value may be computed according to a different setting, for example according to another or an adapted statistical measure. Hence, following the detection of a myocardial infarction event a different setting may be used for computing any of the reference values, each of the reference values representing a detection parameter to be used for identifying a premature ventricular contraction event.

In one embodiment, the processing arrangement is configured to classify the ventricular contraction event which is currently assessed as a premature ventricular contraction event if the at least one discrimination metric value deviates from the first reference value by more than a first margin and/or deviates from the second reference value by more than a second margin. For classifying a currently assessed ventricular contraction event as a premature ventricular contraction event, the processing arrangement compares one or multiple computed discrimination metric values to one or multiple reference values relating to prior ventricular contraction events and/or subsequent ventricular contraction events. If it is found that the one or the multiple discrimination metric values differ from associated reference values, this is interpreted to indicate that the waveform of the currently assessed ventricular contraction event is abnormal in that it differs from a waveform of a regular ventricular contraction event, and accordingly the ventricular contraction event may be classified as a premature ventricular contraction event (wherein potentially further conditions may be taken into account).

For example, the processing arrangement is configured to determine the first margin based on a percentage value of the first reference value and/or to determine the second margin based on a percentage value of the second reference value. The first reference value and the second reference value are dynamically determined based on a number of prior ventricular contraction events and/or based on a number of subsequent ventricular contraction events. Based on the current value for the first reference value and/or the second reference value the first margin and/or the second margin are set. The percentage value herein may be fixed, e.g. in a range between 1% to 50% or the like.

In one embodiment, said first margin represents a third detection parameter and/or said second margin represents a fourth detection parameter. The first margin and/or the second margin hence represent (each) a detection parameter, which is adapted in response to the occurrence of a myocardial infarction event. For example, in a default state a first value may be used for the first margin respectively the second margin, for example a certain, respective percentage value. In response to the occurrence of a myocardial infarction event the respective margin is adapted such that a second value for the detection parameter is used, for example a second percentage value different than the first value.

In one embodiment the system comprises an external device configured to operate outside of the patient. The implantable medical device is configured to communicate with the external device outside of the patient, wherein the implantable medical device is configured to communicate information relating to identified ventricular contraction events, information relating to myocardial infarction events and/or information relating to identified cardiac arrhythmia events to the external device. Dependent on the amount of processing which is carried out by the implantable medical device, the information communicated to the external device may differ. If the implantable medical device carries out the entire processing for identifying cardiac arrhythmia events, the implantable medical device may communicate information relating to identified cardiac arrhythmia events to the external device. If the implantable medical device carries out only some of the processing, the implantable medical device may communicate information relating to intermediate processing results to the external device. If the entire processing for identifying cardiac arrhythmia events is carried out by processing equipment outside of the implantable medical device, the implantable medical device may for example communicate only information relating to recorded electrocardiogram signals to the external device, for example within the context of a home monitoring system.

The implantable medical device may, in one embodiment, comprise multiple electrode poles, which for example are aligned along a longitudinal axis and hence are arranged at different axial positions on the implantable medical device. By means of the different electrode poles an electrical coupling to surrounding tissue is established when the implantable medical device is implanted in a patient, such that electrocardiogram signals may be sensed using the different electrode poles.

In another aspect, a method for operating a system for identifying a cardiac arrhythmia event in a patient comprises: sensing, using an arrangement of electrode poles of an implantable medical device, electrocardiogram signals; and processing electrocardiogram signals obtained by the implantable medical device; wherein the processing includes: using a first value of at least one detection parameter for detecting a cardiac arrhythmia event based on said electrocardiogram signals, obtaining information indicative of a myocardial infarction event, in response to obtaining said information, adapting said at least one detection parameter to a second value different than the first value for detecting a cardiac arrhythmia event in a time period subsequent to obtaining said information indicative of a myocardial infarction event.

The advantages and advantageous embodiments described above for the system equally apply also to the method, such that it shall be referred to the above in this respect.

Generally, the implantable medical device may comprise a processing circuitry with may be configured to carry out at least some of the processing steps of the method. This however is not necessary. In one embodiment, the implantable medical device is configured to sense electrocardiogram signals, but is not configured to process electrocardiogram signals by carrying out particular processing steps for identifying cardiac arrhythmia events. In one embodiment, some or all of the processing steps for identifying cardiac arrhythmia events are carried out by a processing circuitry of the implantable medical device.

The various features and advantages of the present invention may be more readily under-stood with reference to the following detailed description and the embodiments shown in the drawings. Herein,
- Fig. 1: shows a schematic drawing of an implantable medical device implanted in a patient;
- Fig. 2: shows a schematic drawing of an embodiment of an implantable medical device comprising an arrangement of electrode poles;
- Fig. 3: shows a schematic drawing of another embodiment of an implantable medical device;
- Fig. 4: shows a ventricular contraction event indicative of a myocardial infarction event;
- Fig. 5: shows a schematic drawing of a time period for identifying a cardiac arrhythmia event following a myocardial infarction event;
- Fig. 6: shows a waveform relating to a premature ventricular contraction event;
- Fig. 7: shows a series of ventricular contraction events; and
- Fig. 8: shows a premature ventricular contraction event within a series of ventricular contraction events.

Subsequently, embodiments of the invention shall be described in detail with reference to the drawings. In the drawings, like reference numerals designate like structural elements.

It is to be noted that the embodiments are not limiting for the invention, but merely represent illustrative examples.

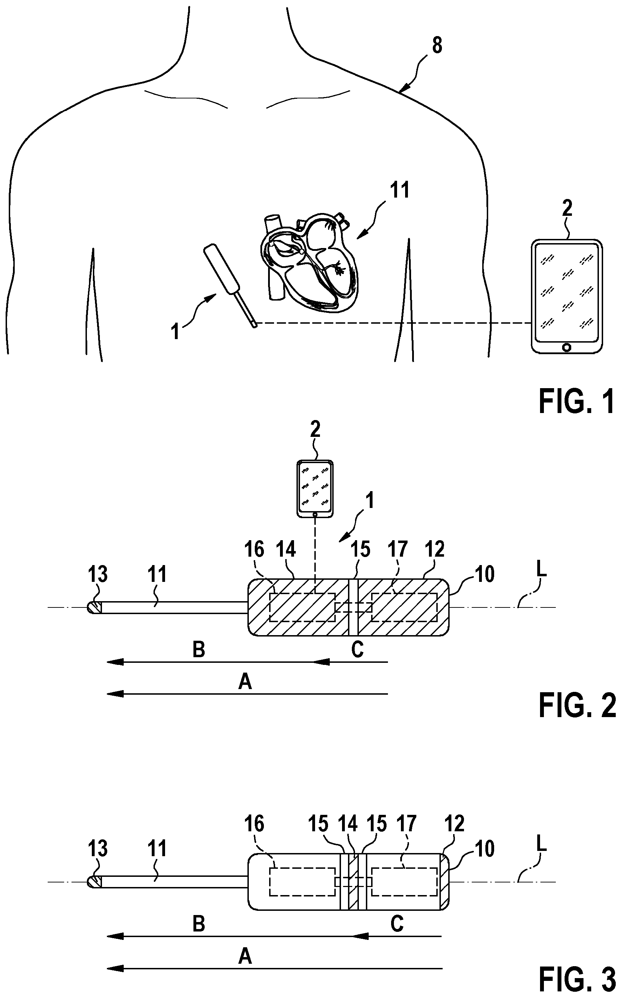

Referring to Fig. 1, in one embodiment a system comprises an implantable medical device 1 implanted (for example subcutaneously) into a patient for serving a therapeutic and/or diagnostic function. The implantable medical device 1 may for example be implanted subcutaneously into a patient P for monitoring cardiac activity of the patient's heart H. The implantable medical device 1, for this, comprises an arrangement of electrode poles which are used to couple to surrounding tissue and to sense electrocardiogram signals originating from the heart H.

The system furthermore comprises an external device 2 external to the patient P and being in communication connection with the implantable medical device 1.

Referring now to Fig. 2, in one embodiment the implantable medical device 1 comprises a housing 10 formed e.g. by different housing segments, the housing 10 enclosing and encapsulating a processing module 16 formed by electronic circuitry and a battery module 17. In particular, a first housing segment may receive and enclose the processing module 16, whereas a second housing segment receives and encloses the battery module 17. Another housing segment longitudinally extends from the first and second housing segments and forms a header portion 11 having reduced cross-sectional dimensions with respect to the other housing segments.

In the embodiment of Fig. 2, a first electrode pole 12 is formed by the housing segment enclosing the battery module 17, a second electrode pole 13 is arranged at a far end of the housing segment forming the header portion 11, and (optional) a third electrode pole 14 is formed by the housing segment enclosing the processing module 16. The implantable medical device 1 with its housing 10 generally extends along a longitudinal axis L, the electrode poles 12, 13, 14 being aligned along the longitudinal axis L and being axially displaced with respect to one another along the longitudinal axis L. The electrode poles 12, 13, 14 herein are electrically separated from one another, an electrically insulating segment 15 being arranged in between the electrode poles 12, 14 formed on the main housing portion and the header portion 11 formed by the housing segment separating the electrode pole 13 from the other two electrode poles 12, 14.

In the embodiment of Fig. 2, the electrode poles 12, 13, 14 may be formed by portions of the housing 10 itself, the housing 10 being made for example from an electrically conductive material, in particular a metal material. By exposing portions of the housing 10 towards the outside, the electrode poles 12, 13, 14 are formed and may electrically contact with surrounding tissue in order to establish a coupling between the electrode poles 12, 13, 14 to the surrounding tissue.

Referring now to Fig. 3, in another embodiment the first electrode pole 12 is formed at an end of a housing segment of the housing 10 encapsulating the battery module 17, whereas the (optional) electrode pole 14 is formed by an electrode element which is electrically insulated from other portions of the housing 10 by the electrically insulating segments 15. For example, a multilayered pole element may be employed for forming the electrode pole 14, as it is described for example in EP 3 278 836 B1. The electrode pole 13 again is formed at a far end of the housing segment forming the header portion 11.

In any of the embodiments of Fig. 2 and 3, using the arrangement of electrode poles 12, 13, 14, electrocardiogram signals may be received and processed by the processing module 16. Based on the processing, a communication with an external device 2 may be established, for example to transmit alert messages to the external device 2 for example within the context of a home monitoring system for monitoring a physiological state of the patient P.

The different electrode poles 12, 13, 14 herein define signal reception vectors A, B, C by means of which electrocardiogram signals may be received using pairs of associated electrode poles 12, 13, 14. In particular, a first signal reception vector A is formed between the first electrode pole 12 and the second electrode pole 13, a second signal reception vector B is formed between the third electrode pole 14 and the first second electrode pole 13, and a third signal reception vector C is formed between the first electrode pole 12 and the third electrode pole 14. As the first electrode pole 12 and the second electrode pole 13 are arranged at opposite ends of the housing 10, the associated signal reception vector A is longer than the other two signal reception vectors B, C.

The different electrode poles 12, 13, 14 form different pairs of electrode poles 12, 13, 14 spanning different signal reception vectors A, B, C. By means of the different signal reception vectors A, B, C different electrocardiogram signals may be received and may be processed in a multi-channel processing.

It shall be noted herein that the implantable medical device 1 may be a monitoring device (as schematically shown in Figs. 2 and 3), a pacemaker device, a defibrillator device or any other implantable medical device configured for implantation into a patient P. The instant text in particular is not limited to a monitoring device configured for implantation outside of a patient's heart H.

A system comprising an implantable medical device 1 and an external device 2 as described herein shall generally be configured to identify cardiac arrhythmia events, such as ventricular tachycardia events, ventricular fibrillation events and/or premature ventricular contraction events.

Herein, for identifying a cardiac arrhythmia event one or multiple detection parameters shall be employed according to a first setting in a default state. In case of a myocardial infarction event, the settings for one or multiple of the detection parameters are adapted such that upon the occurrence of a myocardial infarction event a different setting for detecting a cardiac arrhythmia event is employed.

In particular, it can be assumed that in an acute phase following the occurrence of a myocardial infarction event a patient is faced with an increased risk of cardiac arrhythmias, such cardiac arrhythmias being potentially hazardous for the patient following a myocardial infarction. Hence, in a time period following a myocardial infarction event it shall be made sure that cardiac arrhythmia events are reliably detected, potentially at the expense of an increased number of false positive classifications.

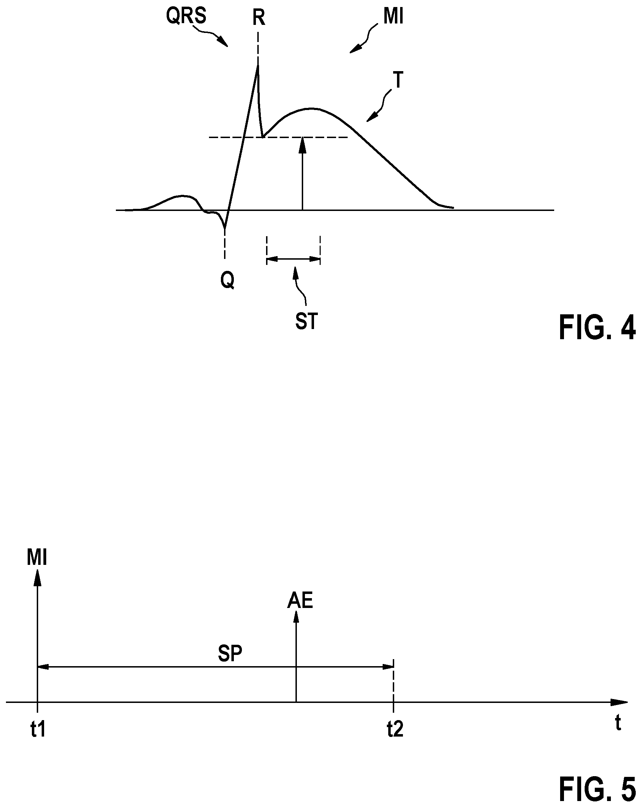

Referring now to Fig. 4, in one embodiment the system is configured to automatically detect a myocardial infarction event MI based on sensed electrocardiogram signals. In particular, a processing arrangement of the system, in particular a processing module 16 of the implantable medical device 1 potentially in cooperation with a processing circuitry of an external device 2, may be configured to process sensed electrocardiogram signals in order to identify QRS wave forms relating to ventricular contraction events. According to such QRS wave forms a myocardial infarction event MI may be identified based on a so-called ST segment elevation corresponding to an increased segment elevation above a baseline in a so-called ST segment subsequent to a QRS complex and a following T wave. Based on an increased ST segment elevation a myocardial infarction event MI may be identified, information relating to a myocardial infarction event MI may be output to an external device 2, and settings may be adapted for a further processing of sensed electrocardiogram signals following the detection of a myocardial infarction event MI.

Alternatively or in addition, information relating to a myocardial infarction event may be input to the system, for example to the external device 2, the information subsequently being communicated for example to the implantable medical device 1. In particular, a myocardial infarction event may be identified by clinical personnel by diagnostic tools outside of the system, upon which information relating to an identified myocardial infarction event may be entered into the system by clinical personnel.

Whereas in a default state - without the presence of a myocardial infarction event MI - default settings for detecting a cardiac arrhythmia event may be employed by the system (in particular the processing module 16 of the implantable medical device 1), such settings may be adapted following the identification of a myocardial infarction event MI such that different settings are employed for detecting a cardiac arrhythmia event following the identification of a myocardial infarction event MI.

Referring now to Fig. 5, at a time t1 a myocardial infarction event MI is identified, either automatically by the system, in particular the implantable medical device 1, or by manual user input. Upon the identification of the myocardial infarction event MI at time t1, the system is configured to adapt settings for detecting cardiac arrhythmia events AE in a time period SP (of increased sensitivity) between a time t1 and a time t2 following the identification of the myocardial infarction event MI, such that within the time period SP different settings are employed to detect cardiac arrhythmias.

Following the time period SP, the system may automatically revert back to its original, default settings, such that upon lapse of the time period SP again the default settings for detection parameters for detecting cardiac arrhythmia events are employed.

The system, in particular the implantable medical device 1 with its processing module 16, may be configured to detect cardiac arrhythmia events such as ventricular tachycardia events, ventricular fibrillation events and/or premature ventricular contraction events.

For example, a ventricular tachycardia may be detected by assessing electrocardiogram signals, wherein a ventricular tachycardia is identified if a sequence of a predefined number of cardiac beats exhibits a heart rate exceeding a predefined threshold.

In a default setting, the predefined number of cardiac beats may for example lie in a range between 6 to 20 cardiac beats, for example at 8 cardiac beats. The heart rate threshold may for example lie at a value of 200 bpm. That is, if for a sequence of heartbeats according to the predefined number of cardiac beats the heart rate threshold is exceeded, e.g. for example for 8 cardiac beats a heart rate is determined which is above 200 bpm, it is concluded for a ventricular tachycardia event.

In case a myocardial infarction event MI is detected, such settings for detecting a ventricular tachycardia within the time period SP may be adapted. For example, the setting for the predefined number of cardiac beats may be adapted. Alternatively or in addition, the setting for the heart rate threshold may be adapted. For example the setting for the predefined number of cardiac beats may be adapted to a lower number, for example a number in between 2 to 10 cardiac beats, for example 4 cardiac beats. The heart rate threshold may be adapted to a lower heart rate value, for example in a range between 80 bpm and 140 bpm, for example 90 bpm, 100 bpm, 110 bpm or 120 bpm. Following the detection of a myocardial infarction event MI, a ventricular tachycardia hence is identified with an increased sensitivity. For example, if the predefined number of cardiac beats is set to 4 and the heart rate threshold is set to 120 bpm, a ventricular tachycardia is detected following a myocardial infarction event MI already if for 4 cardiac beats a heart rate exceeding 120 bpm is detected.

In another example, premature ventricular contraction events may be detected based on a processing of electrocardiogram signals, for example based on a timing and/or based on a processing of morphology parameters of ventricular contraction events, wherein settings for detection parameters may be adapted following the identification of a myocardial infarction event MI.

Generally, it is characteristic for a premature ventricular contraction event that the premature ventricular contraction event comes at a premature, short timing distance after a prior ventricular contraction event and is followed by a comparatively lengthy pause before another, subsequent ventricular contraction event occurs. In addition, a premature ventricular contraction event generally exhibits a waveform which in its morphology substantially differs from the waveform of a regular ventricular contraction event.

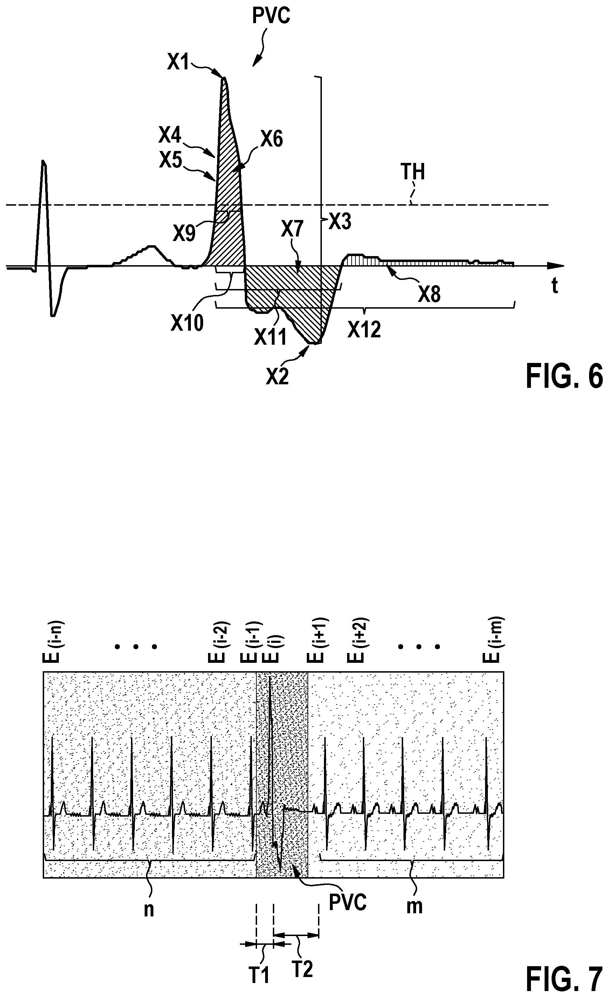

Referring now to Fig. 6, a premature ventricular contraction event PVC comprises a morphology different than a regular ventricular contraction event of a regular sine rhythm of the patient's heart H. The morphology herein may be characterized by certain discrimination metrics, such as a maximum positive amplitude X1, a maximum negative amplitude X2, a maximum peak-to-peak amplitude X3, a maximum slope value X4, an maximum value of the second derivative X5, an area X6 under a positive R peak prior to a first zero-crossing X6, an area X7 under the curve between a first zero-crossing and a second zero-crossing, an area X8 under the curve between the second zero-crossing and a third zero-crossing, a time duration X9 between an upward crossing and a downward crossing of a ventricular detection threshold TH, a time duration X10 between the upward crossing of the ventricular detection threshold TH and the first zero-crossing, a time duration X11 between the upward crossing of the ventricular detection threshold TH and the second zero-crossing, and/or a time duration X12 between the upward crossing of the ventricular detection threshold TH and a third zero-crossing.

For a particular ventricular contraction waveform in question, values for all or some of the discrimination metrics may be computed and may be assessed in order to identify whether an abnormal morphology potentially indicative of a premature ventricular contraction event is present.

To classify at least one ventricular contraction event as a premature ventricular contraction event, at least one discrimination metric value X1... X12 is computed. The at least one discrimination metric value is then compared to at least one reference value, and based on the comparison the ventricular contraction event in question is classified as a premature ventricular contraction waveform (or not).

Referring now to Fig. 7, reference values may in particular be determined according to a number n of prior ventricular contraction events E(i-n).. E(i-1) and/or a number m of subsequent ventricular contraction events E(i+1)...E(i+m).

In particular, for a specific discrimination metric value, such as the maximum positive amplitude X1 or the area X6 under the positive R peak prior to the first zero-crossing, an associated first reference value may be computed based on the associated discrimination metric for the number n of prior ventricular contraction events E(i-n)...E(i-1). Alternatively or in addition, a second reference value may be computed based on the associated discrimination metric for the number m of subsequent ventricular contraction events E(i+1)...E(i+m).

The particular reference value may in particular be computed according to a statistical measure by applying a statistical analysis. For example, the particular reference value may correspond to a mean value, a standard deviation value, a coefficient of variation, a Shannon entropy value, an exponential moving average value, a median value, a percentile value, a skew value, a kurtosis value, or a root mean square value relating to the particular discrimination metric X1...X12.

For example, if the maximum positive amplitude X1 is assessed as the discrimination metric, the first reference value may be determined by averaging the maximum positive amplitude values of the n prior ventricular contraction events E(i-n)...E(i-1), and the second reference value may be determined by averaging the maximum positive amplitude values of the m subsequent ventricular contraction events E(1+1)...E(i+m).

Herein, settings for computing the particular reference value may be adapted following the identification of a myocardial infarction event MI. In particular, in a default state a first setting for computing a particular reference value may be employed. Following the identification of a myocardial infarction event MI, the setting may be adapted such that the particular reference value is computed according to a different statistical measure or by using an adapted processing for computing a statistical measure.

Making use of the reference values, the particular ventricular contraction event in question may be classified as a premature ventricular contraction event if for example a particular discrimination metric value as computed for the ventricular contraction event differs by more than a certain margin from the respective reference value. For example, if both a first reference value relating to prior ventricular contraction events E(i-n)...E(i-1) and a second reference value relating to subsequent ventricular contraction events E(i+1)...E(i+m) is taken into account, a ventricular contraction event may be classified as a premature ventricular contraction event if the discrimination metric value differs by more than a first margin from the first reference value and by more than a second margin from the second reference value.

If multiple discrimination metric values relating to different discrimination metrics are computed, multiple different first reference values and/or or multiple different second reference values may be taken into account, the different reference values relating to the different discrimination metrics. Herein, the ventricular contraction event may be classified as a premature ventricular contraction event if at least for a subset of the discrimination metric values an associated set of conditions is fulfilled. For example, it may be found for a premature ventricular contraction event if for two out of three of the discrimination metric values it is found that the particular discrimination metric value differs from an associated reference value by more than a certain margin.

The margin, in each case, may be for example computed based on a percentage of the particular reference value, wherein the percentage may be fixed or may be dynamically adapted during operation of the system.

Each margin represents a detection parameter for detecting a premature ventricular contraction. The setting for the particular margin may be adapted following the identification of a myocardial infarction event MI, such that a different setting for the respective margin, for example a different percentage value, may be used following the identification of a myocardial infarction event MI in comparison to a default state.

In the example of Fig. 7, for the ventricular contraction event E(i) it for example is found that based on one or multiple discrimination metric values relating to one or multiple different discrimination metrics a substantial deviation of the morphology in comparison to prior ventricular events E(in)... E(i-1) and/or to subsequent ventricular events E(i+1)... E(i+m) is present, such that the ventricular contraction event E(i) is classified as a premature ventricular contraction event PVC.

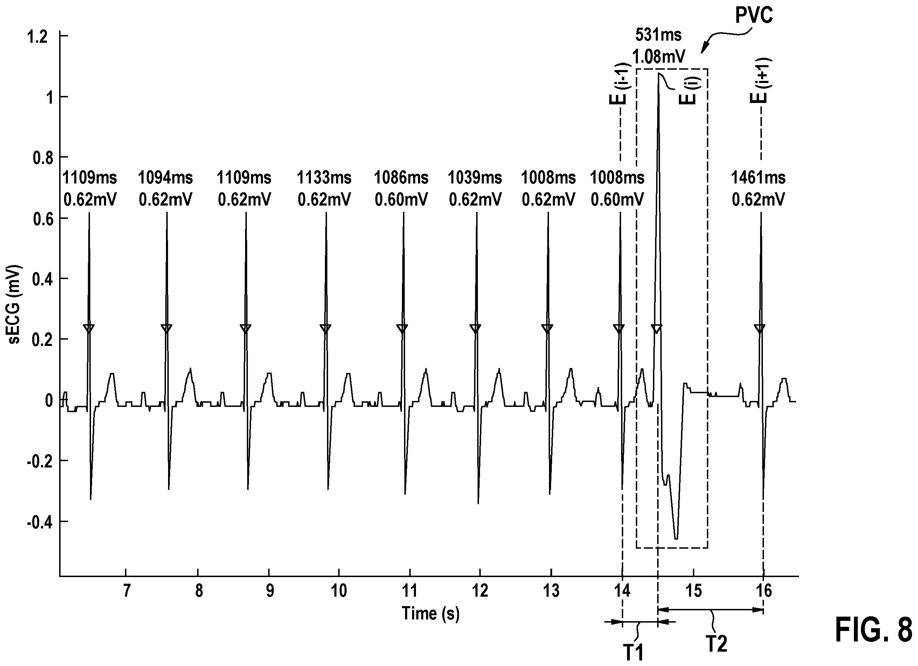

Generally, a premature ventricular contraction event PVC not only substantially differs in the morphology of the associated waveform, but also in the timing from a regular contraction event. Hence, in addition or alternatively to assessing a contraction's morphology, it may be assessed whether a timing distance T1 of the ventricular contraction event E(i) is smaller than a first timing threshold, hence indicating that the ventricular contraction event E(i) occurs prematurely with respect to a prior ventricular contraction event E(i-1), as indicated in Fig. 8. In addition, it may be assessed whether a timing distance T2 of the ventricular contraction event E(i) is larger than a second timing threshold, hence indicating that after the ventricular contraction event E(i) a substantial pause occurs, longer than the length of a regular heartbeat, as visible in Fig. 8.

The timing thresholds represent detection parameters. Herein, following the identification of a myocardial infarction event MI a different setting for a particular timing threshold may be employed in comparison to a default state, such that following the identification of a myocardial infarction event MI a setting for the particular timing threshold is adapted.

### List of reference numerals

- 1: Implantable medical device
- 10: Housing
- 11: Header portion
- 12: First electrode pole
- 13: Second electrode pole
- 14: Third electrode pole
- 15: Electrically insulating segment
- 16: Processing module
- 17: Battery module
- 2: External device
- A, B, C: Signal reception vector
- E(i-n)...E(i+m): Ventricular contraction event
- H: Heart
- i: Current ventricular contraction event (beat)
- L: Longitudinal axis
- n: Prior ventricular contraction events (beats)
- m: Subsequent ventricular contraction events (beats)
- MI: Myocardial infarction event
- P: Patient
- PVC: Premature ventricular contraction event
- QRS: QRS complex
- Q: Q wave
- R: R wave
- SP: Time period (of increased sensitivity)
- ST: ST segment
- T: T wave
- T1: Timing distance
- T2: Timing distance
- TH: Ventricular detection threshold
- X1...X12: Discrimination metric value

## Claims

1. A system for identifying a cardiac arrhythmia event (AE) in a patient (P), the system comprising:
an implantable medical device (1) for sensing electrocardiogram signals, the implantable medical device (1) comprising an arrangement of electrode poles (12, 13) configured to sense electrocardiogram signals; and
a processing arrangement for processing electrocardiogram signals obtained by the implantable medical device (1);
wherein the processing arrangement is configured to use a first setting of at least one detection parameter for detecting a cardiac arrhythmia event (AE) based on said electrocardiogram signals,
obtain information indicative of a myocardial infarction event (MI), and
**characterized in that**
in response to obtaining said information, adapt said at least one detection parameter to a second setting different than the first setting for detecting a cardiac arrhythmia event (AE) in a time period (SP) subsequent to obtaining said information indicative of a myocardial infarction event (MI).

2. The system according to claim 1, wherein the implantable medical device (1) comprises a processing module (16) forming at least part of the processing arrangement.

3. The system according to claim 1 or 2, wherein the processing arrangement is configured, for obtaining said information indicative of a myocardial infarction event (MI), to detect a myocardial infarction event (MI) based on said electrocardiogram signals.

4. The system according to one of claims 1 to 3, wherein the processing arrangement is configured to adapt said at least one detection parameter in response to the myocardial infarction event (MI) to detect a cardiac arrhythmia event (AE) with an increased sensitivity in said time period (SP) subsequent to obtaining said information indicative of a myocardial infarction event (MI).

5. The system according to one of the preceding claims, wherein the processing arrangement is configured to detect, as a cardiac arrhythmia event (AE), at least one of a ventricular tachycardia event, a ventricular fibrillation event, a bradycardia event, an asystolic event, or a premature ventricular contraction event (PVC).

6. The system according to one of the preceding claims, wherein the at least one detection parameter includes at least one of a threshold for a heart rate value, a number of heart beats for determining a heart rate value, a timing threshold indicative of a timing between successive hearts beats, or an amplitude threshold.

7. The system according to one of the preceding claims, wherein the processing arrangement is configured to adapt said at least one detection parameter back to the first setting for detecting a cardiac arrhythmia event (AE) upon lapse of said time period (SP).

8. The system according to one of the preceding claims, wherein said time period (SP) is in a range between 1 hour and 30 days.

9. The system according to one of the preceding claims, wherein the processing arrangement is configured to identify at least one ventricular contraction event (E(i)) based on said electrocardiogram signals and to classify said ventricular contraction event (E(i)) as a cardiac arrhythmia event (AE) based on said at least one detection parameter.

10. The system according to one of the preceding claims, wherein the processing arrangement is configured to classify at least one ventricular contraction event (E(i)) as a premature ventricular contraction event (PVC) based on a first timing distance (T1) between the ventricular contraction event (E(i)) and an immediately prior ventricular contraction event (E(i-1)) and/or a second timing distance (T2) between the ventricular contraction event (E(i)) and an immediately subsequent ventricular contraction event (E(i+1)).

11. The system according to claim 10, wherein the processing arrangement is configured to evaluate, for said classifying, whether the first timing distance (T1) between the ventricular contraction event (E(i)) and an immediately prior ventricular contraction event (E(i-1)) is smaller than a first timing threshold, said first timing threshold representing a first detection parameter, and/or whether the second timing distance (T2) between the ventricular contraction event (E(i)) and an immediately subsequent ventricular contraction event (E(i+1)) is larger than a second timing threshold, said second timing threshold representing a second detection parameter.

12. The system according to one of the preceding claims, wherein the processing arrangement is configured to compute at least one discrimination metric value (X1...X12) for at least one ventricular contraction event (E(i)), to compare said at least one discrimination metric value (X1...X12) to at least one of a first reference value computed based on a first number (n) of prior ventricular contraction events (E(i-n)... E(i-1)) and a second reference value computed based on a second number (n) of subsequent ventricular contraction events (E(i-1)...E(i+m)), and to classify said ventricular contraction event (E(i)) as a premature ventricular contraction event (PVC) based on said comparison.

13. The system according to claim 12, wherein the processing arrangement is configured to compute said first reference value based on a first statistical measure relating to said first number (n) of prior ventricular contraction events (E(i-n)... E(i-1)) and/or compute said second reference value based on a second statistical measure relating to said second number (n) of subsequent ventricular contraction events (E(i-1)...E(i+m)).

14. The system according to claim 12 or 13, wherein the processing arrangement is configured to classify said ventricular contraction event (E(i)) as a premature ventricular contraction event (PVC) if said at least one discrimination metric value (X1... X12) deviates from said first reference value by more than a first margin and/or deviates from said second reference value by more than a second margin, wherein said first margin represents a third detection parameter and/or said second margin represents a fourth detection parameter.

## Patentansprüche

1. System zum Identifizieren eines Herzrhythmusstörungsereignisses (AE) bei einem Patienten (P), wobei das System umfasst:
eine implantierbare medizinische Vorrichtung (1) zum Erfassen von Elektrokardiogrammsignalen, wobei die implantierbare medizinische Vorrichtung (1) eine Anordnung von Elektrodenpolen (12, 13) umfasst, die dazu ausgebildet ist, Elektrokardiogrammsignale zu erfassen; und
eine Verarbeitungseinrichtung zum Verarbeiten von durch die implantierbare medizinische Vorrichtung (1) erhaltenen Elektrokardiogrammsignalen;
wobei die Verarbeitungseinrichtung dazu ausgebildet ist,
eine erste Einstellung mindestens eines Detektionsparameters zum Detektieren eines Herzrhythmusstörungsereignisses (AE) auf der Grundlage der Elektrokardiogrammsignale zu verwenden,
Informationen zu erhalten, die auf ein Myokardinfarktereignis (MI) hinweisen, und **dadurch gekennzeichnet, dass**
die Verarbeitungseinrichtung als Reaktion auf das Erhalten der Informationen den mindestens einen Detektionsparameter auf eine zweite Einstellung anpasst, die von der ersten Einstellung verschieden ist, zum Detektieren eines Herzrhythmusstörungsereignisses (AE) in einem Zeitraum (SP) nach dem Erhalten der Informationen, die auf ein Myokardinfarktereignis (MI) hinweisen.

2. System nach Anspruch 1, wobei die implantierbare medizinische Vorrichtung (1) ein Verarbeitungsmodul (16) umfasst, das zumindest einen Teil der Verarbeitungseinrichtung bildet.

3. System nach Anspruch 1 oder 2, wobei die Verarbeitungseinrichtung dazu ausgebildet ist, zum Erhalten der Informationen, die auf ein Myokardinfarktereignis (MI) hinweisen, um ein Myokardinfarktereignis (MI) auf der Grundlage der Elektrokardiogrammsignale zu detektieren.

4. System nach einem der Ansprüche 1 bis 3, wobei die Verarbeitungseinrichtung dazu ausgebildet ist, den mindestens einen Detektionsparameter als Reaktion auf das Myokardinfarktereignis (MI) anzupassen, um ein Herzrhythmusstörungsereignis (AE) mit einer erhöhten Empfindlichkeit in dem Zeitraum (SP) nach dem Erhalten der Informationen, die auf ein Myokardinfarktereignis (MI) hinweisen, zu detektieren.

5. System nach einem der vorhergehenden Ansprüche, wobei die Verarbeitungseinrichtung dazu ausgebildet ist, als Herzrhythmusstörungsereignis (AE) mindestens eines von einem ventrikulären Tachykardieereignis, einem ventrikulären Fibrillationsereignis, einem Bradykardieereignis, einem Asystolieereignis oder einem vorzeitigen ventrikulären Kontraktionsereignis (PVC) zu detektieren.

6. System nach einem der vorhergehenden Ansprüche, wobei der mindestens eine Detektionsparameter mindestens eines von einem Schwellenwert für einen Herzfrequenzwert, einer Anzahl von Herzschlägen zur Bestimmung eines Herzfrequenzwerts, einem Zeitschwellenwert, der auf einen zeitlichen Abstand zwischen aufeinanderfolgenden Herzschlägen hinweist, oder einem Amplitudenschwellenwert umfasst.

7. System nach einem der vorhergehenden Ansprüche, wobei die Verarbeitungseinrichtung dazu ausgebildet ist, den mindestens einen Detektionsparameter nach Ablauf des Zeitraums (SP) auf die erste Einstellung zum Detektieren eines Herzrhythmusstörungsereignisses (AE) zurück anzupassen.

8. System nach einem der vorhergehenden Ansprüche, wobei der Zeitraum (SP) in einem Bereich zwischen 1 Stunde und 30 Tagen liegt.

9. System nach einem der vorhergehenden Ansprüche, wobei die Verarbeitungseinrichtung dazu ausgebildet ist, mindestens ein ventrikuläres Kontraktionsereignis (E(i)) auf der Grundlage der Elektrokardiogrammsignale zu identifizieren und das ventrikuläre Kontraktionsereignis (E(i)) auf der Grundlage des mindestens einen Detektionsparameters als Herzrhythmusstörungsereignis (AE) zu klassifizieren.

10. System nach einem der vorhergehenden Ansprüche, wobei die Verarbeitungseinrichtung dazu ausgebildet ist, mindestens ein ventrikuläres Kontraktionsereignis (E(i)) als vorzeitiges ventrikuläres Kontraktionsereignis (PVC) auf der Grundlage eines ersten Zeitabstands (T1) zwischen dem ventrikulären Kontraktionsereignis (E(i)) und einem unmittelbar vorhergehenden ventrikulären Kontraktionsereignis (E(i-1)) und/oder eines zweiten Zeitabstands (T2) zwischen dem ventrikulären Kontraktionsereignis (E(i)) und einem unmittelbar nachfolgenden ventrikulären Kontraktionsereignis (E(i+1)) zu klassifizieren.

11. System nach Anspruch 10, wobei die Verarbeitungseinrichtung dazu ausgebildet ist, für das Klassifizieren auszuwerten, ob der erste Zeitabstand (T1) zwischen dem ventrikulären Kontraktionsereignis (E(i)) und einem unmittelbar vorhergehenden ventrikulären Kontraktionsereignis (E(i-1)) kleiner ist als ein erster Zeitschwellenwert, wobei der erste Zeitschwellenwert einen ersten Detektionsparameter darstellt, und/oder ob der zweite Zeitabstand (T2) zwischen dem ventrikulären Kontraktionsereignis (E(i)) und einem unmittelbar nachfolgenden ventrikulären Kontraktionsereignis (E(i+1)) größer ist als ein zweiter Zeitschwellenwert, wobei der zweite Zeitschwellenwert einen zweiten Detektionsparameter darstellt.

12. System nach einem der vorhergehenden Ansprüche, wobei die Verarbeitungseinrichtung dazu ausgebildet ist, mindestens einen Diskriminierungsmetrikwert (X1...X12) für mindestens ein ventrikuläres Kontraktionsereignis (E(i)) zu berechnen, den mindestens einen Diskriminierungsmetrikwert (X1...X12) mit mindestens einem von einem ersten Referenzwert, der auf der Grundlage einer ersten Anzahl (n) vorhergehender ventrikulärer Kontraktionsereignisse (E(i-n)...E(i-1)) berechnet wird, und einem zweiten Referenzwert, der auf der Grundlage einer zweiten Anzahl (n) nachfolgender ventrikulärer Kontraktionsereignisse (E(i-1)...E(i+m)) berechnet wird, zu vergleichen, und das ventrikuläre Kontraktionsereignis (E(i)) auf der Grundlage des Vergleichs als vorzeitiges ventrikuläres Kontraktionsereignis (PVC) zu klassifizieren.

13. System nach Anspruch 12, wobei die Verarbeitungseinrichtung dazu ausgebildet ist, den ersten Referenzwert auf der Grundlage eines ersten statistischen Maßes bezüglich der ersten Anzahl (n) vorhergehender ventrikulärer Kontraktionsereignisse (E(i-n)...E(i-1)) zu berechnen und/oder den zweiten Referenzwert auf der Grundlage eines zweiten statistischen Maßes bezüglich der zweiten Anzahl (n) nachfolgender ventrikulärer Kontraktionsereignisse (E(i-1)...E(i+m)) zu berechnen.

14. System nach Anspruch 12 oder 13, wobei die Verarbeitungseinrichtung dazu ausgebildet ist, das ventrikuläre Kontraktionsereignis (E(i)) als vorzeitiges ventrikuläres Kontraktionsereignis (PVC) zu klassifizieren, wenn der mindestens eine Diskriminierungsmetrikwert (X1...X12) von dem ersten Referenzwert um mehr als einen ersten Toleranzwert abweicht und/oder von dem zweiten Referenzwert um mehr als einen zweiten Toleranzwert abweicht, wobei der erste Toleranzwert einen dritten Detektionsparameter darstellt und/oder der zweite Toleranzwert einen vierten Detektionsparameter darstellt..

## Revendications

1. Système d'identification d'un événement d'arythmie cardiaque (AE) chez un patient (P), le système comprenant :
un dispositif médical implantable (1) destiné à détecter des signaux d'électrocardiogramme, le dispositif médical implantable (1) comprenant un agencement de pôles d'électrode (12, 13) configuré pour détecter des signaux d'électrocardiogramme ; et
un agencement de traitement destiné à traiter les signaux d'électrocardiogramme obtenus par le dispositif médical implantable (1) ;
dans lequel l'agencement de traitement est configuré pour
utiliser un premier réglage d'au moins un paramètre de détection pour détecter un événement d'arythmie cardiaque (AE) en se basant sur lesdits signaux d'électrocardiogramme,
obtenir des informations indiquant un événement d'infarctus du myocarde (MI), et **caractérisé en ce que**
en réponse à l'obtention desdites informations, adapter ledit au moins un paramètre de détection à un second réglage différent du premier réglage pour détecter un événement d'arythmie cardiaque (AE) dans une période de temps (SP) consécutive à l'obtention desdites informations indiquant un événement d'infarctus du myocarde (MI).

2. Système selon la revendication 1, dans lequel le dispositif médical implantable (1) comprend un module de traitement (16) formant au moins une partie de l'agencement de traitement.

3. Système selon la revendication 1 ou 2, dans lequel l'agencement de traitement est configuré, pour obtenir lesdites informations indiquant un événement d'infarctus du myocarde (MI), pour détecter un événement d'infarctus du myocarde (MI) en se basant sur lesdits signaux d'électrocardiogramme.

4. Système selon l'une des revendications 1 à 3, dans lequel l'agencement de traitement est configuré pour adapter ledit au moins un paramètre de détection en réponse à l'événement d'infarctus du myocarde (MI) pour détecter un événement d'arythmie cardiaque (AE) avec une sensibilité augmentée dans ladite période de temps (SP) consécutive à l'obtention desdites informations indiquant un événement d'infarctus du myocarde (MI).

5. Système selon l'une des revendications précédentes, dans lequel l'agencement de traitement est configuré pour détecter, en tant qu'événement d'arythmie cardiaque (AE), au moins un parmi un événement de tachycardie ventriculaire, un événement de fibrillation ventriculaire, un événement de bradycardie, un événement asystolique, ou un événement d'extrasystole ventriculaire (PVC).

6. Système selon l'une des revendications précédentes, dans lequel l'au moins un paramètre de détection inclut au moins parmi un seuil pour une valeur de fréquence cardiaque, un nombre de battements de cœur pour déterminer une valeur de fréquence cardiaque, un seuil temporel indiquant une caractéristique temporelle entre des battements de cœur successifs, ou un seuil d'amplitude.

7. Système selon l'une des revendications précédentes, dans lequel l'agencement de traitement est configuré pour adapter ledit au moins un paramètre de détection en retour au premier réglage pour détecter un événement d'arythmie cardiaque (AE) lors de l'écoulement de ladite période de temps (SP).

8. Système selon l'une des revendications précédentes, dans lequel ladite période de temps (SP) est dans une plage entre 1 heure et 30 jours.

9. Système selon l'une des revendications précédentes, dans lequel l'agencement de traitement est configuré pour identifier au moins un événement de contraction ventriculaire (E(i)) en se basant sur lesdits signaux d'électrocardiogramme et pour classer ledit événement de contraction ventriculaire (E(i)) en tant qu'événement d'arythmie cardiaque (AE) en se basant sur ledit au moins un paramètre de détection.

10. Système selon l'une des revendications précédentes, dans lequel l'agencement de traitement est configuré pour classer au moins un événement de contraction ventriculaire (E(i)) en tant qu'événement d'extrasystole ventriculaire (PVC) en se basant sur une première distance temporelle (T1) entre l'événement de contraction ventriculaire (E(i)) et un événement de contraction ventriculaire immédiatement antérieur (E(i-1)) et/ou une seconde distance temporelle (T2) entre l'événement de contraction ventriculaire (E(i)) et un événement de contraction ventriculaire immédiatement consécutif (E(i+1)).

11. Système selon la revendication 10, dans lequel l'agencement de traitement est configuré pour évaluer, pour ladite classification, si la première distance temporelle (T1) entre l'événement de contraction ventriculaire (E(i)) et un événement de contraction ventriculaire immédiatement antérieur (E(i-1)) est inférieur à un premier seuil temporel, ledit premier seuil temporel représentant un premier paramètre de détection, et/ou si la seconde distance temporelle (T2) entre l'événement de contraction ventriculaire (E(i)) est un événement de contraction ventriculaire immédiatement consécutif (E(i+1)) est supérieure à un second seuil temporel, ledit second seuil temporel représentant un deuxième paramètre de détection.

12. Système selon l'une des revendications précédentes, dans lequel l'agencement de traitement est configuré pour calculer au moins une valeur de métrique de discrimination (X1...X12) pour au moins un événement de contraction ventriculaire (E(i)), pour comparer ladite au moins une valeur de métrique de discrimination (X1...X12) à au moins une parmi première valeur de référence calculée en se basant sur un premier nombre (n) d'événements de contraction ventriculaire antérieurs (E(i-n)...E(i-1)) et une seconde valeur de référence calculée en se basant sur un second nombre (n) d'événements de contraction ventriculaire consécutifs (E(i-1)...E(i+m)), et pour classer ledit événement de contraction ventriculaire (E(i)) en tant qu'événement d'extrasystole ventriculaire (PVC) en se basant sur ladite comparaison.

13. Système selon la revendication 12, dans lequel l'agencement de traitement est configuré pour calculer ladite première valeur de référence en se basant sur une première mesure statistique relative audit premier nombre (n) d'événements de contraction ventriculaire antérieurs (E(i-n)...E(i-1))et/ou calculer ladite seconde valeur de référence en se basant sur une seconde mesure statistique liée audit second nombre (n) d'événements de contraction ventriculaire consécutifs (E(i-1)...E(i+m)).

14. Système selon la revendication 12 ou 13, dans lequel l'agencement de traitement est configuré pour classer ledit événement de contraction ventriculaire (E(i)) en tant qu'événement d'extrasystole ventriculaire (PVC) si ladite au moins une valeur de métrique de discrimination (X1...X12) s'écarte de ladite première valeur de référence de plus d'une première marge et/ou s'écarte de ladite seconde valeur de référence de plus d'une seconde marge, dans lequel ladite première marge représente un troisième paramètre de détection et/ou ladite seconde marge représente un quatrième paramètre de détection.
